# EUROPEAN PATENT SPECIFICATION

(11) **EP 3 254 699 B1**
(45) Date of publication and mention of the grant of the patent: **09.08.2023**
(21) Application number: 16746799.2
(22) Date of filing: 29.01.2016
(51) Int. Cl.: A61K 9/14, A61K 9/28, A61P 17/14, A61P 13/08, A61K 47/69, A61K 31/58

(54) **SOLID DISPERSION CONTAINING DUTASTERIDE, AND COMPOSITION CONTAINING SAME**
FESTE DISPERSION MIT DUTASTERID UND ZUSAMMENSETZUNG DAMIT
DISPERSION SOLIDE CONTENANT DU DUTASTÉRIDE ET COMPOSITION LA CONTENANT

(30) Priority: 02.02.2015 KR 20150016251
(43) Date of publication of application: 13.12.2017
(73) Proprietor: Whanin Pharmaceutical. Co., Ltd., Seoul 05809 (KR)
(72) Inventor: AHN, Byung-Nak, Seoul 04170 (KR); MIN, Mi Hong, Bundang-gu Seongnam-si Gyeonggi-do, 13588 (KR); PARK, Jin Hyeong, Suwon-si Gyeonggi-do 16509 (KR)
(74) Representative: Louis Pöhlau Lohrentz
(86) International application number: PCT/KR2016/001035
(87) International publication number: WO 2016/126058

(56) References cited:
- EP-A1- 2 050 436
- KR-A- 20080 077 258
- KR-B1- 100 962 447
- KR-B1- 101 055 412
- US-A1- 2005 096 296
- MIN-SOO KIM: "Influence of hydrophilic additives on the supersaturation and bioavailability of dutasteride-loaded hydroxypropyl- [beta]-cyclodextrin nanostructures", INTERNATIONAL JOURNAL OF NANOMEDICINE, 1 May 2013 (2013-05-01), page 2029, XP055474676, DOI: 10.2147/IJN.S44795
- Min-Soo Kim: "Improved Supersaturation and Oral Absorption of Dutasteride by Amorphous Solid Dispersions In-Hwan Beak a", The Pharmaceutical Society of Japan Chem. Pharm. Bull. Republic of Korea. Received June, 1 January 2012 (2012-01-01), pages 1468-1473, XP055161211, Retrieved from the Internet: URL:https://www.jstage.jst.go.jp/article/c pb/60/11/60_c12-00563/_pdf [retrieved on 2015-01-12]
- V.B Chaudhary ET AL: "Cyclodextrin inclusion complex to enhance solubility of poorly water soluble drugs: a review", International Journal of Pharmaceutical Sciences and Research, 1 January 2013 (2013-01-01), pages 68-76, XP055474792, Retrieved from the Internet: URL:http://ijpsr.com/wp-content/uploads/20 14/10/7-Vol.-4-Issue-1-January-2013-IJPSR- RE-706-Paper-71.pdf [retrieved on 2018-05-14]

## Description

### [TECHNICAL FIELD]

A first present invention relates to a solid dispersion containing dutasteride, more specifically to a solid dispersion containing an inclusion complex which includes dutasteride or a pharmaceutically acceptable salt thereof and cyclodextrin, and at least one selected from the group consisting of a thickener and a surfactant.

A second present invention relates to a pharmaceutical composition containing dutasteride, more specifically to a composition containing the solid dispersion according to the first present invention and a pharmaceutically acceptable additive, formulated in a form for oral administration such as tablets, granules or capsules, etc.

### [BACKGROUND FIELD]

### [Characteristics of a formulation containing dutasteride which is currently available]

Dutasteride is a compound disclosed in US Patent No. 5,565,467. As disclosed in WO 06/099121, dutasteride is a white powder which generally has a melting point of 242-250°C and is an active ingredient (hereinafter, "drug") of 0.5 mg of Avodart (AVODART^{®}) soft capsule which is commercially available as a drug product for treating benign prostate hyperplasia and androgenetic alopecia of adult men (aged between 18 and 50).

The above-mentioned Avodart is a soft gelatin capsule in which 0.5 mg of dutasteride is dissolved in a mixture of mono-di-glyceride of capryl/capric acid and dibutyl hydroxy toluene.

In general, the soft capsule is a formulation which is prepared by encapsulating the active ingredient or a substance made by putting a proper excipient, etc. in the active ingredient with a proper capsule material such as gelatin, etc. having the increased plasticity through polyhydric alcohol such as glycerin or sorbitol to be molded in a fixed shape. The active ingredient in the capsule material is mainly a liquid or a suspension in which the active ingredient is evenly dispersed as fine particles in a liquid excipient.

The soft capsule has the following disadvantages: The soft capsule is bulky to be conveniently taken. It is easily softened at a high temperature, while it may delay the dissolution at a low temperature due to the hardening of gelatin and oil. Additionally, additional production facilities are necessary because the soft capsule is in a liquid state, which causes considerable production costs. Furthermore, a drug in the gelatin soft capsule exists in a liquid state, and thus the drug may be transitioned into the film of the soft capsule as time goes by. In addition, the drug may be leaked because the gelatin film is torn, which may cause an issue in safety to pharmacists and patients due to its sexual hormone-like property.

Therefore, in general, it is preferable to formulate a drug in a solid formulation, if possible.

Solubility of dutasteride is 44 mg/mL in ethanol, 64 mg/mL in methanol and 3 mg/mL in polyethylene glycol 400, and is a poorly soluble drug in water. In the case of administering a drug, the systemic absorption of the drug occurs through a series of steps of disintegration of the drug, dissolution of the drug in an aqueous solution environment and systemic circulation through a cell membrane. However, a poorly soluble drug, for which the above-mentioned dissolution process is not easily performed, has a relatively low bioavailability.

In response thereto, the poorly soluble drug is developed into an injection for direct intravascular administration or into a formulation such as a liquid phase, etc. Avodart, which has been commercially available, adopted the soft capsule which contains the drug as the liquid phase or suspended phase.

### [Solidification means of a formulation containing dutasteride which has been conventionally suggested]

In order to overcome the disadvantages of the soft capsule, the development of a solid formulation was on the rise. Most researches which have been conducted so far suggest a self-emulsifying method for a method for solidifying dutasteride.

As an example thereof, Korean Patent No. 0962447 discloses a solidified formulation which has a similar dissolution rate to Avodart by dissolving dutasteride, which is a poorly soluble drug, together with a water-soluble polymer and a surfactant in an oil to prepare a self-emulsifying composition, granulating the composition using a wet granulation method, and post-mixing the granule with a lubricant, etc.

Additionally, Korean Patent No. 1055412, the improvement patent of Korean Patent No. 0962447, discloses a formulation, characterized by increasing hardness of a formulation containing a dutasteride self-emulsifying composition to reduce a risk of being broken during a preparation process, improving easiness in handling, preparing the formulation through first and second coating steps to control the initial dissolution, and containing an adsorbing agent.

### [Solidification means of a formulation containing dutasteride which is suggested in the present invention]

There is a disadvantage that a poorly soluble drug does not represent a desired bioavailability due to low absorption rate of the drug, or the difference between individuals is severe.

The solubility of the poorly soluble drug may be improved by the modification of the crystal form of the drug, the reduction of the particle size of the drug, the use of a self-emulsifying system, the use of a co-solvent or the use of a surfactant, etc. The inclusion complex is also one of various methods for improving the solubility of the poorly soluble drug. Meanwhile, a proper self-emulsifying system method or an inclusion method is totally different depending on the drugs. Additionally, it is very difficult to significantly improve solubility to the satisfaction level when using a general method without numerous repeated experiments or implementation examples for another drug alone.

Unlike the self-emulsifying system, which has been conventionally suggested as the means for solidifying a formulation containing dutasteride, the present invention is to include dutasteride in a gamma-cyclodextrin and prepare the same as a solid dispersion, thereby improving the solubility of dutasteride.

International Journal of Nanomedicine, 2013:8, 2029-2035 discloses influence of hydrophilic additives on the supersaturation and bioavailability of dutasteride-loaded hydroxypropyl-β-cyclodextrin nanostructures.

Chem. Pharm. Bull. 60(11)1468-1473 (2021) discloses improved supersaturation and oral absorption of dutasteride by amorphous solid dispersions.

Chaudhary et al., IJPSR, 2013; Vol. 4(1): 68-76, discloses cyclodextrin inclusion complex to enhance solubility of poorly water soluble drugs.

### [DETAILED DESCRIPTION OF INVENTION]

### [TECHNICAL TASK]

Korean Patent No. 0962447 does not include *in vivo* pharmacokinetic profile (PK profile), but merely discloses a comparative material on dissolution with respect to Avodart soft capsule.

The self-emulsifying emulsion commonly contains a high content of a surfactant and thus may irritate the gastrointestinal tract. Furthermore, the surfactant may be diluted by gastrointestinal fluids upon *in vivo* administration. The dilution of the surfactant lowers the solubilizing ability of the drug, which may result in the reprecipitation tendency of the drug (Journal of Pharmacy and Alternative Medicine, vol 1 p2012, Basics of Self Micro Emulsifying Drug Delivery System). It is predicted that the formulation disclosed in the prior art patent has the low absorption of dutasteride due to the dilution of the self-emulsifying composition by body fluids upon administration into the body. In other words, there is doubt whether satisfactory solubility of dutasteride is achieved in the body.

In addition upon reviewing the examples of Korean Patent No. 1055412, the compressed tablet containing 0.5 mg, which is the single dose for dutasteride, weighs about 500 mg. Since the corresponding product further performs first and second coatings, it has the significantly greater weight than a tablet containing finasteride, which is 5-alpha reductase inhibitor with the same original efficacy. In other words, said product is relatively difficult to be swallowed for elderly patients with benign prostatic hyperplasia.

The technical task of the present invention is to improve the above-mentioned problems which the prior art has.

### [MEANS FOR ACHIEVING TASK]

The present invention achieved the above task with the following means:
(1) A solid formulation for oral administration comprising a solid dispersion, wherein the solid dispersion comprises a dutasteride inclusion complex; and at least one selected from the group consisting of a thickener and a surfactant, characterized in that the dutasteride inclusion complex contains dutasteride and gamma-cyclodextrin in a weight ratio of 1:1-1:300, and that the weight ratio of dutasteride to the thickener and surfactant is 1:0.05-1/1:0.05-4.
(2) The solid dispersion according to (1) above, characterized in that the thickener is at least one selected from the group consisting of polyvinyl pyrrolidone, polyethylene oxide and gum.
(3) The solid dispersion according to (1) or (2) above, characterized in that the thickener is polyvinyl pyrrolidone.
(4) The solid dispersion according to any one of (1) to (3) above, characterized in that the surfactant is at least one selected from the group consisting of polyethylene glycol almond glyceride, polyethylene glycol caprlic/capric glyceride, lauryl macrogol glyceride, stearoyl macrogol glyceride, tocopheryl polyethylene glycol succinic acid, poloxamer and diethylene glycol monoethyl ether.
(5) The solid dispersion according to any one of (1) to (4) above, characterized in that the surfactant is stearoyl macrogol glyceride.
(6) The solid dispersion according to any one of (1) to (5) above, characterized by further containing a pharmaceutically acceptable additive.
(7) A pharmaceutical composition, characterized by containing the solid dispersion according to any one of (1) to (6) above and a pharmaceutically acceptable additive.

### [EFFECT OF INVENTION]

According to one embodiment of the present invention, it is possible to overcome the disadvantage of an Avodart soft capsule formulation and the solid preparation which utilizes conventional self-emulsifying system.

Especially, according to one embodiment of the present invention, it is possible to prepare a solid formulation. Additionally, the solubility of dutasteride for water is effectively improved, and thus improved bioavailability is achieved upon oral administration. Also, the formulation has a smaller weight or volume than the existing formulation, and thus convenience in taking the formulation is excellent.

### [BRIEF DESCRIPTION OF DRAWINGS]

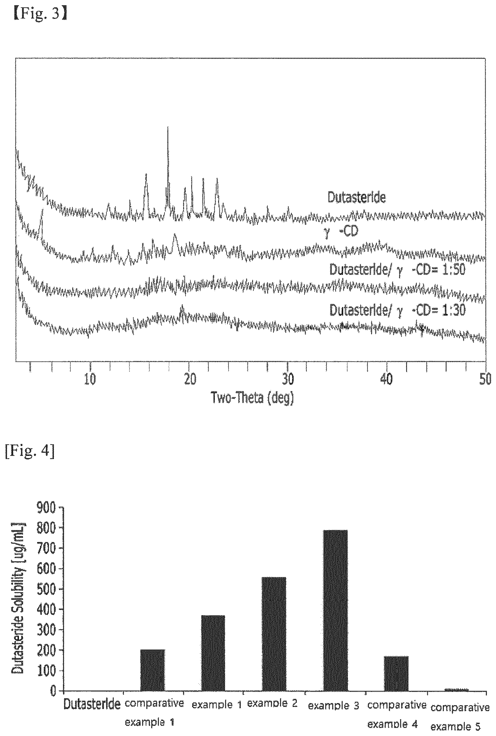

Fig. 1 represents a difference in solubility of an inclusion complex according to the weight ratio of dutasteride and gamma-cyclodextrin.
Fig. 2 represents a difference in crystallinity of dutasteride in the inclusion complexes according to the weight ratios of dutasteride and gamma-cyclodextrin using a differential scanning calorimetry (DSC).
Fig. 3 represents a difference in crystallinity of dutasteride in the inclusion complexes according to the weight ratios of dutasteride and gamma-cyclodextrin using a powder X-ray diffraction (PXRD).
Fig. 4 represents a difference in the degree of solubilization of dutasteride in the solid dispersions of Examples 1 to 3 according to the present invention; the inclusion complex of dutasteride and gamma-cyclodextrin (Comparative Example 1); a simple physical mixture having the same composition as in the example (Comparative Example 4); and a general solid dispersion excluding gamma-cyclodextrin (Comparative Example 5).
   Specifically, dutasteride in Fig. 4 refers to the raw material of dutasteride itself. Comparative Example 1 refers to the inclusion complex in which the weight ratio of dutasteride:gamma-cyclodextrin is 1:100. Example 1 refers to the solid dispersion in which the weight ratio of dutasteride:gamma-cyclodextrin:polyvinyl pyrrolidone is 1:100:0.4. Example 2 refers to the solid dispersion in which the weight ratio of dutasteride:gamma-cyclodextrin:gelucire is 1:100:0.4. Example 3 refers to the solid dispersion in which the weight ratio of dutasteride:gamma-cyclodextrin:polyvinyl pyrrolidone:gelucire is 1 :100:0.4:0.4. Comparative Example 4 refers to a simple mixture in which the weight ratio of dutasteride:gamma-cyclodextrin:polyvinyl pyrrolidone:gelucire is 1:100:0.4:0.4. Comparative Example 5 refers to the solid dispersion in which the weight ratio of dutasteride:polyvinyl pyrrolidone:gelucire is 1 :0.4:0.4.

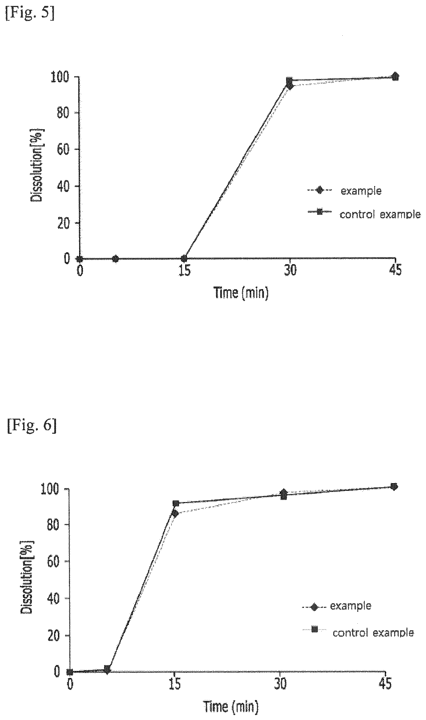

Fig. 5 compares the dissolution rate over the time between the tablet (Example) according to the present invention and Avodart soft capsule (Control Example) under the condition where a test was done using 450 ml of 0.1 N HCL solution containing pepsin for the first 25 minutes, and a dissolution test was done by applying 450 ml of 0.1N HCL solution containing 4% (w/v) SLS at 25 minute.
Fig. 6 compares the dissolution rate over the time between the tablet (Example) according to the present invention and Avodart soft capsule (Control Example) under the condition of the solution at pH 1.2 (2% SLS), 900 ml.

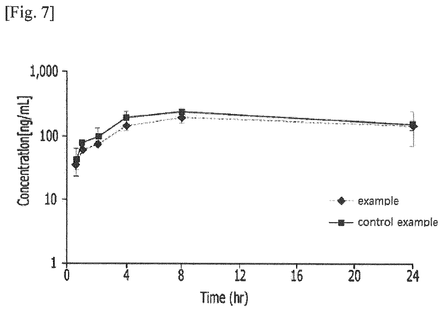

Fig. 7 compares the blood concentration profiles of dutasteride over the time between the solid dispersion (Example) according to the present invention and Avodart soft capsule content (Control Example) after oral administration in rats.

### [BEST MODE FOR EMBODIMENT OF INVENTION]

Dutasteride mentioned herein includes dutasteride and a pharmaceutically acceptable salt thereof.

### [Inclusion complex]

An inclusion complex refers to a sort of a molecular compound in which a certain material molecule (guest molecule) is enclosed by another molecule (host molecule). This kind of inclusion technique may be used in stabilizing drugs, crystallizing drugs in the oil phase, masking bad taste, and solubilizing poorly soluble drugs. For example, when chloramphenicol, ibuprofen, barbituric acid derivatives, etc., are included in beta-cyclodextrin having 6-10Å diameter which is the host molecule, their solubility and bioavailability are known to be improved.

In order to select a proper host molecule, it is necessary to undergo various repeated experiments in consideration of the characteristics of the guest molecule and the objective to be achieved through the inclusion. Since even if a certain host molecule is preferable to a specific guest molecule, it can not be generally preferable to other guest molecules.

The present invention selects gamma-cyclodextrin as the most preferable host molecule after analyzing the results of numerous repeated experiments.

The cyclodextrin inclusion complex has an advantage in simplicity of a preparation process and efficiency of costs, etc. Additionally, since cyclodextrin, cyclic oligosaccharide of alpha-di-glycopyranose, has hydrophobic cavity and hydrophilic exterior, it takes an advantage to form inclusion complex with poorly soluble drug.

For decades, cyclodextrin has been developed into the type of various derivatives. Representative host molecules are alpha-cyclodextrin, beta-cyclodextrin, hydroxypropyl-beta-cyclodextrin, gamma-cyclodextrin or sulfobutyl ether-beta-cyclodextrin, etc.

Among them, an example of including dutasteride in hydroxypropyl-beta-cyclodextrin using a supercritical fluid method is disclosed in the prior art (Int J Nanomedicine, 2013, 8:2029-2039). Meanwhile, since related facilities of cyclodextrin inclusion complexes by the supercritical fluid method have not been commercialized yet, there is a limit in practice for manufacturing cyclodextrin inclusion complexes into a product. The corresponding document introduces that a composition in which the ratio of a drug, hydroxypropyl-beta-cyclodextrin and hydroxypropyl methyl cellulose is about 1:26.6:13.3 is a formulation having the most increased solubility; however, the maximum solubility thereof is merely 47 µg/mL. This degree of solubility is not satisfactory in consideration of the task to be achieved by the present invention. Additionally, when hydroxypropyl methyl cellulose exists in a relatively great amount compared to the drug in the above formulation, the release of the drug in the aqueous solution could be slow due to the corresponding amount of polymer.

The present invention is characterized by forming inclusion complex using gamma-cyclodextrin among the cyclodextrins.

When compared with other cyclodextrins, gamma-cyclodextrin has a low interaction for a phospholipid membrane and can be used for oral and injection materials. Also, the water solubility of gamma-cyclodextrin is about two times higher than that of hydroxypropyl-beta-cyclodextrin and beta-cyclodextrin, and thus about 20% gamma-cyclodextrin-containing aquous solution could be prepared. For this reason, the small volume of solvent could be used to form the inclusion complex between dutasteride and gamma-cyclodextrin., and commercialization thereof was expected to be much efficiently performed. Furthermore, gamma-cyclodextrin inclusion complex has improved the stability of dutasteride.

When dutasteride is included using gamma-cyclodextrin as the host molecule, the weight ratio of gamma-cyclodextrin is 1-300 with respect to dutasteride.

When the weight ratio of gamma-cyclodextrin is less than 1 with respect to dutasteride, the inclusion complex of dutasteride is not made sufficiently, and thus there is little effect of increasing the solubility. When the weight ratio exceeds 300, cyclodextrin exists at a high concentration, leading to aggregation between cyclodextrins, thereby reducing the effect of increasing the drug solubility (International Journal of Pharmaceutics, 2010 mar 15; 387 (1-2): 199-208, Self-assembled cyclodextrin aggregates and nanoparticles), and tablet becomes biggerto reduce the convenience of taking the same.

Especially, the weight ratio of gamma-cyclodextrin is more preferably over 20 and 200 or less, and most preferably over 30 and 100 or less with respect to dutasteride, in terms of effect to be achieved by the present invention.

As the inclusion method, a solvent evaporation method, a spray drying method or a kneading technique, etc. may be used. Additionally, whether the drug is included into cyclodextrin or not may be confirmed through a powder XRD (PXRD), and whether the physical properties of the drug are improved or not may be confirmed by solubility and dissolution test.

### [Solid dispersion]

A solid dispersion is a system where drug particles are dispersed in a solid matrix carrier having excellent solubility. The solid dispersion may widen the surface area of the drug particle by reducing the drug particle size. Additionally, the drug is converted into an amorphous form during the process of making the solid dispersion and thus may exist partially or entirely as an amorphous state. Solid dispersion not only enhances the drug solubility but also is effective in storage stability by controlling the tendency of the amorphous drug to be a crystalline form with a low free energy.

The examples of a typical carrier of the solid dispersion include, but not limited to, water-soluble polymers, i.e., hydroxypropyl methyl cellulose (HPMC), polyethylene glycol (PEG), polyvinyl pyrrolidone (PVP), polyvinyl alcohol (PVA), hydroxypropyl cellulose (HPC), cellulose polymer, poly(meth)acrylate or a mixture thereof, etc. The present invention is characterized by using a material as a carrier which could be utilized as a thickener.

The reason therefore is that surprisingly, it was found that the use of a thickener as the carrier when preparing a solid dispersion containing dutasteride improved the solubilization of dutasteride. The extract cause therefor has not been revealed, but it is assumed that water is pulled around the drug by the thickener which increases the viscosity, and thus the solubilization of the drug would be improved. In general, in the case of hydroxylpropyl cellulose, etc., which is the carrier widely used in the solid dispersion, there was no effect of increasing the solubility of the inclusion complex of dutasteride and gamma-cyclodextrin. However, in the case of polyvinyl pyrrolidone, etc., which is one of the thickeners, it was found that it remarkably increased the solubility of the inclusion complex.

Meanwhile, the thickener refers to a material which can resist flowability in the liquid phase and is also called a viscosity controlling agent. Materials which can be utilized pharmaceutically as the thickener are well known.

In the present invention, any of components which are well known as the thickener may be introduced in consideration of the effect aimed to be achieved by the present invention, and at least one selected from the group consisting of polyvinyl pyrrolidone, polyethylene oxide and gum is preferable. Especially, polyvinyl pyrrolidone among them is the most preferable.

Polyvinyl pyrrolidone is a polymer having the molecular weight of about 2,500-3,000,000 g/mole and has good solubility in water.

Meanwhile, it is generally known that when the content of polyvinyl pyrrolidone in the solid dispersions is higher than that of the drug, its solubility and release rate are shown to be enhanced. As an example thereof, Korean Patent Laid-Open No. 2013-0069484 describes that when the rate of polyvinyl pyrrolidone (referred to as a water-soluble polymer in the corresponding specification) is increased in a solid dispersion containing celecoxib, the solubility of celecoxib is increased. However, the present invention is characterized in that the weight ratio of the thickener such as polyvinyl pyrrolidone to dutasteride is low, 0.05-1. Specifically, since the drug is the amorphous form in solid dispersion, the dissolution rate tends to increase. So it may be expected by referring to the function of crystallinity. The present invention containing the gamma-cyclodextrin inclusion complex can maximize the solubility of the poorly soluble drug without causing any problems such as drug release delay due to excess amount of polymer, unlike the previously known tendency.

Meanwhile, the present invention found that when a surfactant, instead of the thickener or together with the thickener, is added, a remarkable effect was shown in terms of solubility of dutasteride. An exact cause therefor has not been found yet, but it is assumed that the surfactant has excellent accessibility both to water and the inclusion complex of the drug according to the present invention due to its amphiphilic property and thus would increase the solubility.

The surfactant refers to a material for lowering the interfacial tension when dissolving in water or aqueous solution, and materials which can be utilized pharmaceutically as the surfactant are well known.

Especially, in consideration of the effect to be achieved by the present invention, at least one selected from the group consisting of polyethylene glycol almond glyceride, polyethylene glycol caprlic/capric glyceride, lauryl macrogol glyceride, stearoyl macrogol glyceride, tocopheryl polyethylene glycol succinic acid, poloxamer, diethylene glycol monoethyl ether, is preferable as the surfactant. Stearoyl macrogol glyceride is more preferable.

One embodiment of the solid dispersion according to the present invention contains the inclusion complex; and at least one selected from a thickener and a surfactant according to the present invention. The weight ratio of dutasteride with respect to the thickener is 1:0.05-1, and to the surfactant is 1:0.05-4. Especially, among the embodiments of the solid dispersion according to the present invention, the weight ratio of dutasteride to the thickener and surfactant is 1:0.05-1/1:0.05-4 in the case of the solid dispersion containing dutasteride, the thickener and the surfactant.

When the weight ratio of the thickener is less than 0.05, it was extremely difficult to confirm the effect of increasing the solubility by the thickener. When the weight ratio of the thickener is greater than 1, the viscosity becomes higher, and thus the effect of increasing the solubility by cyclodextrin might be reduced.

When the weight ratio of the surfactant is less than 0.05, the effect of increasing the solubility was extremely poor. When the weight ratio of the surfactant is greater than 4, since a high content of surfactant is contained, the above-mentioned problems of the prior art would also occur.

The solid dispersion according to the present invention may further mix other pharmaceutically acceptable additives which can be appropriately selected by a skilled person in the art within the scope of not inhibiting the effect aimed to be achieved by the present invention.

### [Pharmaceutical composition]

A pharmaceutical composition according to the present invention contains a solid dispersion according to any one of claims 1 to 6 and a pharmaceutically acceptable additive formulated in a solid formulation for oral administration. Especially, the pharmaceutical composition may be formulated in the form of tablets, granules or capsules using any formulation techniques.

### [A method for preparing a formulation for oral administration]

The above formulation may be prepared through the steps of (a) dissolving dutasteride in lower alcohols having 1 to 6 carbon atoms, preferably in ethanol, dissolving gamma-cyclodextrin and at least one selected from the group consisting of a thickener and a surfactant in water, and mixing the two liquids; (b) granulating the mixture by a drying method under reduced pressure, a spray drying method or a freeze drying method; and (c) mixing the granule and a pharmaceutically acceptable additive.

Especially, when propylene glycol is added to the step (a), the solubility of dutasteride for the mixture of ethanol and water may be improved, and thus is preferable in terms of physical stability of the mixture.

Also, the step (a) may further mix a pharmaceutically acceptable additive.

Hereinafter, the present invention will be explained in more detail through the following examples.

### [FORM FOR EMBODIMENT OF THE INVENTION]

### [EXPERIMENTAL EXAMPLE 1] Comparison of inclusion complexes in terms of solubilization level according to the type of cyclodextrin

Alpha-cyclodextrin, beta-cyclodextrin, gamma-cyclodextrin and hydroxypropyl-beta-cyclodextrin were dissolved in water, respectively, and dutasteride was dissolved in the same amount of ethanol. Then, the two liquids were mixed to form an inclusion complex. In this case, the weight ratio of dutasteride and cyclodextrin was 1:100. The inclusion complex of cyclodextrin and dutasteride in the form of powder was obtained from the mixture solution of ethanol and distilled water through a drying process, followed by suspension in water at the supersaturation concentration of 1 mg/ml. The solubility was measured with HPLC after 0.45 um filtering. The analysis conditions and the result of solubility are as shown below.

### Analysis conditions

Column: Zobrax SB-Phenyl (150 mm x 3.0 mm, 3.5 um) or a column equivalent thereto
Mobile phase: Mixture liquid of acetonitrile and purified water (55:45, v/v)
Wavelength of detection: UV, 240 nm
Amount of injection: 50 uL
Flow rate: 0.5 mL/min

### Table 1

**[Table 1]**

| Type of cyclodextrin | Solubility of dutasteride |
|---|---|
| Alpha-cyclodextrin | 2 ug/ml |
| Beta-cyclodextrin | 55 ug/ml |
| Gamma-cyclodextrin | 201 ug/ml |
| Hydroxypropyl-beta-cyclodextrin | 61 ug/ml |

As shown in Table 1 above, the inclusion of dutasteride with gamma-cyclodextrin was the most excellent in terms of solubility.

### [EXPERIMENTAL EXAMPLE 2] Comparison of inclusion complexes in terms of solubility according to the ratio of gamma-cyclodextrin

Gamma-cyclodextrins with different weights were dissolved in water for the experiment as in Experimental Example 1 and the solubility of dutasteride was obtained as in Tables 2 and 3 below.

### Table 2

**[Table 2]**

| | | | | | | |
|---|---|---|---|---|---|---|
| Weight ratio of dutasteride : gamma-cyclodextrin | 1:10 | 1:20 | 1:50 | 1:100 | 1:200 | 1:300 |
| Solubility | 10 ug/mL | 30 ug/mL | 91 ug/mL | 205 ug/mL | 91 ug/mL | 70 ug/mL |

### Table 3

**[Table 3]**

| | | | |
|---|---|---|---|
| Weight ratio of dutasteride : gamma-cyclodextrin | 1:0.5 | 1:500 | 1:600 |
| Solubility | 1 ug/mL | 59 ug/mL | 46 ug/mL |

As shown in the tables above, when the weight ratio of dutasteride and gamma-cyclodextrin is smaller than 1:1, almost no effect of increasing the solubility was observed. Additionally, when the weight ratio of gamma-cyclodextrin is over 300, stability was reduced in the state of aqueous solution and precipitation was occured. In other words, in the solution containing a high content of gamma-cyclodextrin, gamma-cyclodextrins aggregate and precipitate as time goes by, which may lead to reduction of the solubility of dutasteride.

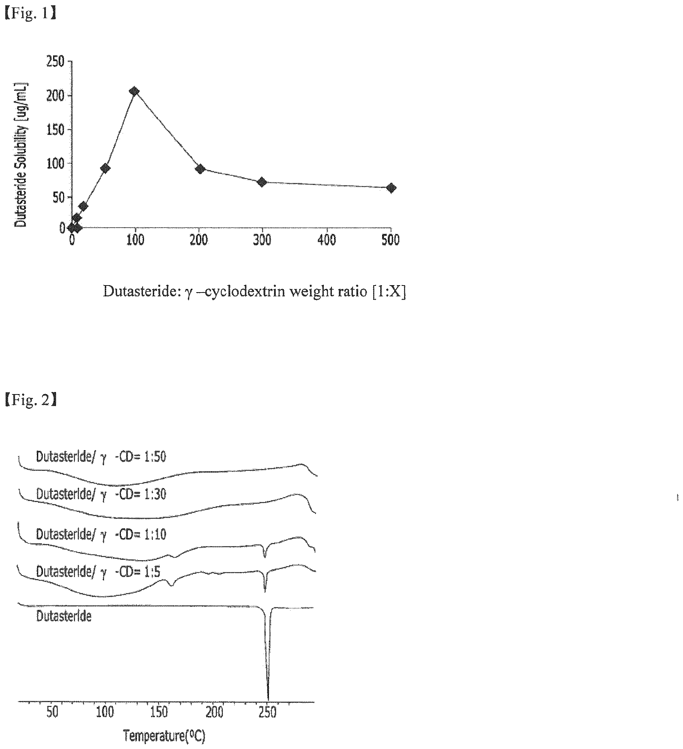

As a result, as shown in Fig. 1, Table 2 and Table 3, in the case of dutasteride, the weight ratio of gamma-cyclodextrin is preferably 1-300, more preferably over 20 and 200 or less, and most preferably over 30 and 100 or less in terms of achievement of the desirable effect according to the present invention.

### [EXPERIMETNAL EXAMPLE 3] Confirmation of crystallinity of inclusion complexes according to the ratio of gamma-cyclodextrin

After preparing dutasteride inclusion complexes as in Experimental Example 1 with the weight ratios of gamma-cyclodextrin to dutasteride of 1:5, 1:10, 1:30 and 1:50, the inclusion complexes were powderized through a drying process and were taken for DSC. As for the inclusion complexes with the weight ratios of 1:30 and 1:50, whether the inclusion complexes were crystallized was confirmed with X-ray diffractometry. The result thereof is as shown in Fig. 2 and Fig. 3.

As shown in Fig. 2 and Fig. 3, it was confirmed that dutasteride was well included in gamma-cyclodextrin, and it was known that a crystalline peak of dutasteride completely disappeared in the weight ratios of 1:30 or more.

### [EXPERIMENTAL EXAMPLE 4] Confirmation of effect of increasing the solubility according to a combination of thickener and surfactant

Gamma-cyclodextrin was dissolved in water, and dutasteride, polyvinyl pyrrolidone and gelucire (macrogol glyceride) were dissolved in the same amount of ethanol. Then, the experiment was performed as in Experimental Example 1 to evaluate the solubility of dutasteride. The result thereof is as shown in Table 4 below.

### Table 4

**[Table 4]**

| Name of component | Compara tive Example 1 | Example 1 | Example 2 | Example 3 | Example 4 | Example 5 | Compara tive Example 2 | Compara tive Example 3 |
|---|---|---|---|---|---|---|---|---|
| Dutasteride | 1.0 mg | 1.0 mg | 1.0 mg | 1.0 mg | 1.0 mg | 1.0 mg | 1.0 mg | 1.0 mg |
| Gamma-cyclodextrin | 100 mg | 100 mg | 100 mg | 100 mg | 100 mg | 100 mg | 100 mg | 100 mg |
| Polyvinyl pyrrolidone | - | 0.4 mg | - | 0.4 mg | 0.05 mg | 1.0 mg | 2.0 mg | 0.01 mg |
| Gelucire | - | - | 0.4 mg | 0.4 mg | 0.05 mg | 1.0 mg | 2.0 mg | 0.01 mg |
| Solubility | 200 ug/mL | 370 ug/mL | 550 ug/mL | 780 ug/mL | 280 ug/mL | 430 ug/mL | 230 ug/mL | 215 ug/mL |

As shown in Table 4, when the dutasteride inclusion complex formed the solid dispersion with polyvinyl pyrrolidone (Example 1) or gelucire (Example 2) or polyvinyl pyrrolidone and gelucire (Examples 3 to 5), the solubility thereof was remarkably excellent compared to dutasteride (solubility of dutasteride, not detected) or dutasteride inclusion complex (Comparative Example 1). Meanwhile, when the weight ratio of the thickener and surfactant deviates from the range of the present invention (Comparative Examples 2 and 3), the increase of the solubility thereof was not greatly remarkable.

### [EXPERIMENTAL EXAMPLE 5] Other comparative experiments

In order to examine the excellent effect of a composition according to the present invention, the solubility was measured as in Experimental Example 1 for various compositions as shown in Table 5 below.

Comparative Example 4 weighed the corresponding amounts of dutasteride, gamma-cyclodextrin, polyvinyl pyrrolidone and gelucire to simply mix them.

Comparative Example 5 used the same preparation method as in Example 3 without using gamma-cyclodextrin.

Comparative Example 6 used the same preparation method as in Comparative Example 4 without using gamma-cyclodextrin.

### Table 5

**[Table 5]**

| Name of component | Example 3 | Example 6 | Comparative Example 4 | Comparative Example 5 | Comparative Example 6 |
|---|---|---|---|---|---|
| Dutasteride | 1.0 mg | 1.0 mg | 1.0 mg | 1.0 mg | 1.0 mg |
| Gamma-cyclodextrin | 100 mg | 100 mg | 100 mg | - | - |
| Polyvinyl pyrrolidone | 0.4 mg | - | 0.4 mg | 0.4 mg | 0.4 mg |
| Polyethyleneoxide | - | 0.1 mg | - | - | - |
| Poloxamer | - | - | - | - | 0.4 mg |
| Tocopheryl polyethylene glycol succinic acid | - | 0.3 mg | - | - | - |
| Gelucire | 0.4 mg | - | 0.4 mg | 0.4 mg | - |
| Classification | Solid dispersion | Solid dispersion | Simple mixture | Solid dispersion | Simple mixture |
| Solubility | 780 ug/mL | 506 ug/mL | 170 ug/mL | 10 ug/mL | 10 ug/mL |

As shown in Table 5, the solid dispersion included with gamma-cyclodextrin is excellent in the effect of increasing the solubility compared to a simple mixture or the solid dispersion which is not included with gamma-cyclodextrin.

Additionally, as shown in Experimental Examples 4 and 5, the solid dispersion exhibited the superior effect of increasing the solubility when the thickener is mixed (Example 1), the surfactant is contained (Example 2) or the thickener and the surfactant are included together (Examples 3 to 6), to the inclusion complex itself of gamma-cyclodextrin alone (Comparative example 1). Also, the solubility of dutasteride in the simple mixture (Comparative Example 4) and in the solid dispersion which does not include gamma-cyclodextrin was inferior to the Examples of the present invention (see Fig. 4).

### [EXPERIMENTAL EXAMPLE 6] Confirmation of dissolution of the dutasteride tablet according to the present invention (Control Example: Avodart soft capsule, 0.5 mg of dutasteride)

15g of gamma-cyclodextrin was dissolved in water, and 0.5 g of dutasteride, 0.4 g of polyvinyl pyrrolidone and 0.3 g of gelucire were dissolved in ethanol. Then, the two liquids were mixed to form a solid dispersion. The mixed liquid was sprayed onto 135 g of lactose using a fluidized bed dryer to obtain solid dispersion powders, and 8 g of croscarmellose sodium and 0.8g of Mg stearate were post-mixed with solid dispersion powders.And then the final mixture compressed to prepare 1000 tablets (160 mg) containing 0.5 mg of dutasteride. In order to give a lag time at pH 1.2, the prepared tablets were subject to about 7% film coating by using Opadry OY-C-7000A and ethyl cellulose, and the dissolution thereof was evaluated together with the Control Example (about 600 mg), following the dissolution testing according to Korean Pharmacopeia. The result thereof is as shown in Figs. 5 and 6.

### [EXPERIMENTAL EXAMPLE 7] Confirmation of PK profile of the tablet according to the present invention (Control Example: Avodart soft capsule, 0.5 mg of dutasteride)

15g of gamma-cyclodextrin was dissolved in water, and 0.5 g of dutasteride, 0.3 g of polyvinyl pyrrolidone and 0.2 g of gelucire were dissolved in ethanol. Then, the two liquids were mixed to form a solid dispersion. The solid dispersion of dutasteride was obtained as powder type from the mixed solution of ethanol and water through a freeze drying process, followed by dissolving in water at the concentration of about 0.25 mg/mL. Then, the amount corresponding to about 0.5 mg as dutasteride was administered to 8-week male SD rats using oral zonde. As for the Control Example, the soft capsule film was torn, and the content corresponding to about 0.5 mg as dutasteride was taken for oral administration. The blood was drawn from the SD rat through carotid artery (tubing) after 0.5, 1, 2, 4, 8 and 24 hours, to be analyzed with LC-MS. The PK profile thereof was compared with the Control Example. The result thereof is as shown in Fig. 7.

## Claims

1. A solid formulation for oral administration comprising a solid dispersion, wherein the solid dispersion comprises a dutasteride inclusion complex; and at least one selected from the group consisting of a thickener and a surfactant, **characterized in that** the dutasteride inclusion complex contains dutasteride and gamma-cyclodextrin in a weight ratio of 1:1-1:300, and that the weight ratio of the dutasteride to thickener and surfactant is 1 :0.05-1/1 :0.05-4.

2. The solid formulation according to claim 1, **characterized in that** the thickener is at least one selected from the group consisting of polyvinyl pyrrolidone, polyethylene oxide and gum.

3. The solid formulation according to any one of claims 1 to 2, **characterized in that** the thickener is polyvinyl pyrrolidone.

4. The solid formulation according to any one of claims 1 to 3, **characterized in that** the surfactant is at least one selected from the group consisting of polyethylene glycol almond glyceride, polyethylene glycol caprylic/capric glyceride, lauryl macrogol glyceride, stearoyl macrogol glyceride, tocopheryl polyethylene glycol succinic acid, poloxamer and diethylene glycol monoethyl ether.

5. The solid formulation according to any one of claims 1 to 4, **characterized in that** the surfactant is stearoyl macrogol glyceride.

6. The solid formulation according to any one of claims 1 to 5, **characterized by** further containing a pharmaceutically acceptable additive.

## Patentansprüche

1. Feste Zubereitung für die orale Verabreichung, umfassend eine feste Dispersion, wobei die feste Dispersion einen Dutasterid-Inklusionskomplex umfasst, und mindestens einen Bestandteil aus der Gruppe, die aus einem Verdickungsmittel und einem oberflächenaktiven Mittel besteht, **dadurch gekennzeichnet, dass** der Dutasterid-Inklusionskomplex Dutasterid und gamma-Cyclodextrin in einem Gewichtsverhältnis von 1:1 - 1:300 enthält und dass das Gewichtsverhältnis des Dutasterids zu Verdickungsmittel und oberflächenaktivem Mittel 1:0,05-1/1:0,05-4 beträgt.

2. Feste Zubereitung nach Anspruch 1, **dadurch gekennzeichnet, dass** das Verdickungsmittel mindestens ein Bestandteil ist, der aus der aus Polyvinylpyrrolidon, Polyethylenoxid und Gummen bestehenden Gruppe ausgewählt ist.

3. Feste Zubereitung nach einem der Ansprüche 1 bis 2, **dadurch gekennzeichnet, dass** das Verdickungsmittel Polyvinylpyrrolidon ist.

4. Feste Zubereitung nach einem der Ansprüche 1 bis 3, **dadurch gekennzeichnet, dass** das oberflächenaktive Mittel mindestens ein Bestandteil ist, der aus der aus Polyethylyenglycolmandelsäureglycerid, Polyethylyenglycolcapryl/caprinsäureglycerid, Laurylmacrogolglycerid, Stearoylmacrogolglycerid, Tocopherylpolyethylenglycolbernsteinsäure, Poloxamer und Diethylenglycolmonoethylether bestehenden Gruppe ausgewählt ist.

5. Feste Zubereitung nach einem der Ansprüche 1 bis 4, **dadurch gekennzeichnet, dass** das oberflächenaktive Mittel Stearoylmacrogolglycerid ist.

6. Feste Zubereitung nach einem der Ansprüche 1 bis 5, **dadurch gekennzeichnet, dass** sie zusätzlich ein pharmazeutisch verträgliches Additiv enthält.

## Revendications

1. Une formulation solide pour administration orale comprenant une dispersion solide, dans laquelle la dispersion solide comprend un complexe d'inclusion de dutastéride ; et au moins l'un choisi dans le groupe constitué d'un épaississant et d'un tensioactif, **caractérisée en ce que** le complexe d'inclusion de dutastéride contient du dutastéride et de la gamma-cyclodextrine dans un rapport en poids de 1:1 à 1:300, et **en ce que** le rapport en poids du dutastéride à l'épaississant et au tensioactif est de 1:0,05 à 1/1:0,05-4.

2. La formulation solide selon la revendication 1, **caractérisée en ce que** l'épaississant est au moins un agent choisi dans le groupe constitué de la polyvinylpyrrolidone, le poly(oxyde d'éthylène) et une gomme.

3. La formulation solide selon l'une quelconque des revendications 1 à 2, **caractérisée en ce que** l'épaississant est la polyvinylpyrrolidone.

4. La formulation solide selon l'une quelconque des revendications 1 à 3, **caractérisée en ce que** le tensioactif est au moins l'un choisi dans le groupe constitué du polyéthylèneglycol glycéride d'amande, du polyéthylèneglycol glycéride caprylique/caprique, du lauryl macrogol glycéride, du stéaroyl macrogol glycéride, du de tocophéryl polyéthylèneglycol acide succinique, du poloxamère et de l'éther monoéthylique de diéthylèneglycol.

5. La formulation solide selon l'une quelconque des revendications 1 à 4, **caractérisée en ce que** le tensioactif est le stéaroyl macrogol glycéride.

6. La formulation solide selon l'une quelconque des revendications 1 à 5, **caractérisée en ce qu'**elle contient en outre un additif pharmaceutiquement acceptable.
